# EUROPÄISCHE PATENTSCHRIFT

(11) **EP 2 632 501 B1**
(45) Veröffentlichungstag und Bekanntmachung des Hinweises auf die Patenterteilung: **26.11.2014**
(21) Anmeldenummer: 11773274.3
(22) Anmeldetag: 25.10.2011
(51) Int. Cl.: A61L 15/16, C08G 18/28, C08G 18/10, C08J 9/00, A61L 15/42, A61L 15/26

(54) **HYDROPHILE, ALIPHATISCHE POLYURETHAN-SCHÄUME**
HYDROPHILIC, ALIPHATIC POLYURETHANE FOAMS
MOUSSES POLYURÉTHANNE ALIPHATIQUES HYDROPHILES

(30) Priorität: 27.10.2010 EP 10189041
(43) Veröffentlichungstag der Anmeldung: 04.09.2013
(73) Patentinhaber: Bayer Intellectual Property GmbH, 40789 Monheim am Rhein (DE)
(72) Erfinder: DÖRR, Sebastian, 40593 Düsseldorf (DE); NIESTEN, Meike, 51061 Köln (DE)
(74) Vertreter: BIP Patents
(86) Internationale Anmeldenummer: PCT/EP2011/068588
(87) Internationale Veröffentlichungsnummer: WO 2012/055834

(56) Entgegenhaltungen:
- EP-A1- 2 143 744
- EP-A2- 0 482 467
- WO-A1-02/074826
- DE-A1-102007 048 080

## Beschreibung

Die Erfindung betrifft ein Verfahren zur Herstellung von hydrophilen, aliphatischen Polyurethan-Schäumen. Weitere Gegenstände der Erfindung sind spezielle Zusammensetzungen zur Herstellung der Polyurethan-Schäume nach dem erfindungsgemäßen Verfahren, aus den erfmdungsgemäßen Zusammensetzungen erhältliche Polyurethanschäume, sowie die Verwendung der Polyurethanschäume als Wundauflage, kosmetischer Artikel oder Inkontinenzprodukt.

Verfahren zur Herstellung von hydrophilen, aliphatischen Polyurethan-Schäumen bzw. die Verwendung derartiger Schäume als Wundauflagen sind im Stand der Technik offenbart. So ist in der EP 949 285 A die Reaktion von Polyisocyanaten mit primären Diaminen, niedermolekularen Polyolen und hochmolekularen Polyolen zu derartigen Schäumen beschrieben. Bei dieser Reaktion kann es jedoch dazu kommen, dass erhebliche Teile der isocyanatreaktiven Substanzen nicht umgesetzt werden und dann aus dem hydrophilen Schaum extrahierbar sind.

Aus der GB 1 571 730 ist die Reaktion von Diisocyanaten wie Isophorondiisocyanat (IPDI) und Bis(isocyanatocyclohexyl)methan (HMDI) mit hohem Dampfdruck mit Polyolen zu PolyurethanSchäumen bekannt. Auch hier bleiben jedoch nicht umgesetzte Komponenten zurück. Ferner ist das Arbeiten mit freien, nicht derivatisierten Diisocyanaten aus Sicht der Arbeitshygiene problematisch. In der WO 2004013215 werden ebenfalls leicht flüchtige Diisocyanate umgesetzt.

Die WO 2003/097727, US 5,065,752 und US 5,064,653 beschreiben Schaumbildungsreaktionen von Präpolymeren in Gegenwart von Acrylamid-Acrylsäure-Copolymeren. Diese Produkte sind chemisch nicht angekoppelt und können komplett extrahiert werden, was nicht wünschenswert ist.

In der US 3,903,232 werden Präpolymere mit Polyethern umgesetzt. Auch bei den hier erhaltenen Schäumen besteht die Gefahr des Auftretens nicht angekoppelter Polyole. Die US 5,296,518 beschreibt ebenso die Umsetzung von Präpolymeren mit Polyethern, wobei drei unterschiedliche Polyole eingesetzt werden, was die Wirtschaftlichkeit dieses Verfahrens in Frage stellt. Zudem lässt sich mit dem dort beschriebenen Verfahren nicht sicherstellen, dass keine niedermolekularen Isocyanate im Gemisch verbleiben..

In der PCT-Anmeldung WO 2010/003559 und in den nicht veröffentlichten europäischen Patentanmeldungen mit den Anmeldenummern 10000158.5 und 09015400.6 sind Verfahren zur Herstellung von besonders hydrophilen Polyurethanschäumen beschrieben. Diese Schäume basieren auf aliphatischen Isocyanaten. Im Vergleich zu den bekannten Verfahren zur Herstellung von Polyurethanschäumen, bei denen aromatische Isocyanaten verwendet werden, sind bei den hier beschriebenen Verfahren auf Grund der geringeren Reaktivität der eingesetzten aliphatischen Isocyanate die Reaktionszeiten länger. Um die Systeme aber auf bereits vorhandenen, für schnell reagierende aromatische Prepolymere ausgelegten Anlagen, verarbeiten zu können, ist es notwendig, die Reaktionszeiten der beschriebenen Verfahren zu reduzieren, ohne dass dabei die Schaumeigenschaften negativ beeinflusst werden.

Aufgabe der vorliegenden Erfindung war es daher ein Verfahrens zur Herstellung hydrophiler, aliphatischer Polyurethan-Schäume mit einer im Vergleich zu den im Stand der Technik bekannten Verfahren, reduzierten Reaktionszeit bereit zu stellen.

Die nach dem erfindungsgemäßen Verfahren erhältlichen Polyurethanschäume sollen dabei insbesondere als Bestandteil einer Wundauflage, eines kosmetischen Artikels oder eines Inkontinenzprodukts eingesetzt werden können, und daher nur wenig extrahierbare Bestandteile aufweisen sollen.. Überdies sollten bei deren Herstellung nur Polyisocyanate mit einem niedrigen Dampfdruck, also keine unmodifizierten Diisocyanate, eingesetzt werden. Die hydrophilen, aliphatischen PolyurethanSchäume sollten außerdem ein schnelles und hohes Absorptionsvermögen für physiologische Salzlösung bzw. Wundflüssigkeit aufweisen. Zuletzt sollen Wundauflagen aus den Polyurethan-Schäumen auch zellverträglich, d.h. nicht cytotoxisch sein und sich in der Anwendung der Wundform optimal anpassen.

Diese Aufgabe wird erfindungsgemäß durch ein Verfahren zur Herstellung hydrophiler, aliphatischer Polyurethan-Schäume gelöst, bei dem Zusammensetzungen umfassend
A) isocyanatfunktionelle Präpolymere erhältlich durch Umsetzung von
   A1) niedermolekularen, aliphatischen Diisocyanaten einer Molmasse von 140 bis 278 g/mol mit
   A2) di- bis hexafunktionellen Polyalkylenoxiden einer OH-Zahl von 22,5 bis 112 mg KOH/g und einem Ethylenoxidanteil von 50 bis 100 mol%, bezogen auf die Gesamtmenge der enthaltenen Oxyalkylengruppen,
B) Alkalimetallsalze schwacher anorganischer Säuren,
C) Wasser
D) gegebenenfalls heterocyclische 4-Ring- oder 6-Ring-Oligomere von niedermolekularen, aliphatischen Diisocyanaten mit einer Molmasse von 140 bis 278 g/mol,
E) gegebenenfalls Katalysatoren,
F) gegebenenfalls Tenside,
G) gegebenenfalls ein- oder mehrwertige Alkohole und
H) gegebenenfalls hydrophile Polyisocyanate erhältlich durch Umsetzung von
   H1) niedermolekularen, aliphatischen Diisocyanaten einer Molmasse von 140 bis 278 g/mol und / oder daraus herstellbaren Polyisocyanaten mit einer Isocyanatfunktionalität von 2 bis 6 mit
   H2) monofunktionellen Polyalkylenoxiden einer OH-Zahl von 10 bis 250 und einem Ethylenoxidanteil von 50 bis 100 mol%, bezogen auf die Gesamtmenge der enthaltenen Oxyalkylengruppen,
bereitgestellt, aufgeschäumt und ausgehärtet werden, wobei Alkalimetallsalze schwacher anorganischer Säuren B) verwendet werden, deren korrespondierende freie Säuren in Wasser bei 25 °C einen pK_{S}-Wert von ≥ 4,0 aufweisen.

Für das erfindungsgemäße Verfahren ist eine geringere Reaktionszeit als bei den oben genannten, im Stand der Technik beschriebenen Verfahren zur Herstellung aliphatischer Polyurethanschäume nötig. D.h. die vollständige Umsetzung der einzelnen Komponenten erfolgt hier schneller, so dass das erfindungsgemäße Verfahren auch auf existierenden, für die Herstellung von Schäumen aus schnell reagierende aromatische Prepolymere ausgelegten Anlagen realisiert werden kann. Weiterhin ist trotz der beschleunigten Reaktion eine ausreichende Verarbeitungszeit gegeben, d.h. die Komponenten reagieren nicht so schnell miteinander, dass keine vollständige Vermischung mehr möglich wäre.

Überaschenderweise kann durch Zusatz von organischen Säuren bzw. deren Salzen, wie etwa dem in der WO 2010/003559 genannten Natriumoleat auch bei einer deutlicher Erhöhung der Konzentration in der Zusammensetzung keine geeignete Beschleunigung der Reaktion realisiert werden.

Die nach dem erfindungsgemäßen Verfahren erhältlichen Polyurethan-Schäume weisen darüber hinaus vergleichbar positive chemische und physikalische Eigenschaften wie die aus der WO 2010/003559 bekannten Schäume auf. So ist deren Absorptionsvermögen für physiologische Salzlösung bzw. für Wundflüssigkeit hoch. Außerdem weisen sie nur einen kleinen Anteil an extrahierbaren Bestandteilen auf und sind damit sehr gut zellverträglich.

Das Absorptionsvermögen gegenüber physiologischer Salzlösung beträgt bei den nach dem erfindungsgemäßen Verfahren hergestellten Polyurethan-Schäumen typischerweise 400 bis 2000 % (Masse der aufgenommenen Flüssigkeit bezogen auf die Masse des trockenen Schaums; Bestimmung nach DIN EN 13726-1, Teil 3.2). Im Vergleich zu anderen hydrophilen Schäumen kann daher mit den Polyurethan-Schäumen auch ohne die Verwendung von superabsorbierenden Polymeren eine sehr hohe Absorption von physiologischer Salzlösung erreicht werden. Selbstverständlich ist die Einarbeitung von Superabsorbern aber auch bei den nach dem erfindungsgemäßen Verfahren hergestellten Polyurethan-Schäumen möglich, dies ist jedoch bei diesen Schäumen nicht bevorzugt, da sie bereits eine sehr hohe Absorption aufweisen.

Die nach dem erfindungsgemäßen Verfahren hergestellten Polyurethan-Schäume weisen darüber hinaus eine angenehme Haptik und eine poröse, zumindest teilweise offenzellige Struktur mit miteinander kommunizierenden Zellen auf. Die Rohdichte der Polyurethan-Schäume liegt dabei typischerweise bei 0,01 bis 0,5 g/cm³ (Bestimmung nach DIN 53420). Darüber hinaus weisen die Polyurethan-Schäume eine gute mechanische Festigkeit und hohe Elastizität auf. Typischerweise sind die Werte für die Zugfestigkeit größer als 30 kPa und für die Bruchdehnung größer als 20 % (Bestimmung nach DIN 53504, DIN 53455,).

In Weiterbildung der Erfindung ist vorgesehen, das isocyanatfunktionelle Präpolymere A) mit einem Gewichtsanteil an niedermolekularen, aliphatischen Diisocyanaten mit einer Molmasse von 140 bis 278 g/mol von unter 1,0 Gew.-%, bevorzugt von unter 0,5 Gew.-%, bezogen auf das Präpolymer, verwendet werden. Dieser Gehalt kann durch entsprechend gewählte Einsatzmengen von Diisocyanaten A1) und Polyalkylenoxiden A2) realisiert werden. Bevorzugt ist jedoch der Einsatz des Diisocyanaten A1) im Überschuss bei anschließender, bevorzugt destillativer, Abtrennung nicht umgesetzter Diisocyanate. Die gemäß dieser Ausführungsform erhältlichen Polyurethan-Schäume zeichnen sich durch einen besonders niedrigen Gehalt an potentiell gesundheitsschädlichen extrahierbaren Bestandteilen auf und sind damit besonders gut zellverträglich.

Die Herstellung der isocyanatfunktionellen Präpolymere A) erfolgt typischerweise durch Umsetzung von 1 Mol-Equivalent Polyalkylenoxide A2) mit 1 bis 20 Mol, bevorzugt 1 bis 10 Mol, besonders bevorzugt 5 bis 10 Mol, des Diisocyanats A1). Die Umsetzung erfolgt typischerweise bei 25 bis 140°C, bevorzugt 60 bis 100°C. Wurde mit überschüssigem Diisocyanat gearbeitet, so erfolgt anschließend die Entfernung des Überschusses bevorzugt durch Dünnschichtdestillation.

Vor, während und nach der Reaktion oder destillativen Abtrennung des Diisocyanatüberschusses können saure oder alkylierende Stabilisatoren, wie Benzoylchlorid, Isophthaloylchlorid, Methyltosylat, Chlorpropionsäure, HCl oder Antioxidantien, wie Di-*tert*-butylkresol oder Tocopherol zugesetzt werden.

Der Gehalt Isocyanatgruppen (bestimmt nach DIN-EN ISO 11909) der isocyanatfunktionellen Präpolymere A) beträgt bevorzugt 1,5 bis 4,5 Gew.-%, besonders bevorzugt 1,5 bis 3,5 Gew.% und ganz besonders bevorzugt 1,5 bis 3,0 Gew.-%.

Beispiele für geeignete niedermolekulare, aliphatischen Diisocyanate A1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat, Isophorondiisocyanat, Butylendiisocyanat und Bis(isocyanatocyclohexyl)methan bevorzugt sind.

Gemäß einer besonders bevorzugten Ausführungsform der Erfindung werden als niedermolekulare, aliphatische Diisocyanate A1) ausschließlich Hexamethylendiisocyanat (HDI), Isophorondiisocyanat (IPDI) oder deren Mischungen eingesetzt werden.

Polyalkylenoxide A2) sind bevorzugt Copolymere aus Ethylenoxid und Propylenoxid mit einem Ethylenoxidgehalt, bezogen auf die Gesamtmenge der enthaltenen Oxyalkylengruppen, von 50 bis 100 mol%, bevorzugt von 60 bis 85 mol%, gestartet auf Polyolen oder Aminen. Geeignete Starter dieser Art sind Glycerin, Trimethylolpropan (TMP), Sorbit, Pentaerythrit, Triethanolamin, Ammoniak oder Ethylendiamin.

Die Polyalkylenoxide A2) besitzen typischerweise zahlenmittlere Molekulargewichte von 1000 bis 15000 g/mol, bevorzugt von 3000 bis 8500 g/mol.

Ferner können die Polyalkylenoxide A2) OH-Funktionalitäten von 2 bis 6, bevorzugt von 3 bis 6, besonders bevorzugt von 3 bis 4 aufweisen.

Gemäß einer weiteren bevorzugten Ausführungsform der Erfindung ist vorgesehen, dass Alkalimetallsalze schwacher anorganischer Säuren B) verwendet werden, deren korrespondierende freie Säuren in Wasser bei 25 °C einen pK_{S}-Wert von ≥ 4,0 und ≤ 14,0 aufweisen.

Beispiele besonders geeigneter Alkalimetallsalze schwacher anorganischer Säuren B) sind Kaliumhydroxid, Natriumhydroxid, Kaliumcarbonat, Natriumcarbonat und Natriumhydrogencarbonat, wobei auch beliebige Mischungen dieser Salze mit umfasst sind.

Besonders bevorzugt ist, wenn die Alkalimetallsalze schwacher anorganischer Säuren B) aus der Gruppe Natriumhydroxid, Natriumhydrogencarbonat und Natriumcarbonat ausgewählt sind. In diesem Fall resultiert eine besonders kurze Reaktionszeit.

Das einzusetzende Wasser C) kann als solches, als Kristallwasser eines Salzes, als Lösung in einem dipolar-aprotischen Lösungsmittel oder auch als Emulsion eingesetzt werden. Bevorzugt wird das Wasser als solches oder in einem dipolar-aprotischen Lösungsmittel eingesetzt. Ganz besonders bevorzugt wird das Wasser als solches eingesetzt.

Gegebenenfalls einzusetzende Ring-Oligomere D) sind heterocyclische, 4-Ring- oder 6-Ring-Oligomere von niedermolekularen, aliphatischen Diisocyanaten mit einer Molmasse von 140 bis 278 g/mol wie Isocyanurate, Iminooxadiazindione oder Uretdione der vorgenannten niedermolekularen aliphatischen Diisocyanate. Bevorzugt sind heterocyclische 4-Ring-Oligomere wie Uretdione. Der durch den Einsatz der Ring-Oligomere D) erhöhte Gehalt an Isocyanatgruppen sorgt für ein besseres Aufschäumen, da in der Isocyanat-Wasser-Reaktion mehr CO₂ gebildet wird..

Zur weiteren Beschleunigung der Reaktion können Katalysatoren E) zugesetzt werden. Dabei handelt es sich typischerweise um die dem Fachmann aus der Polyurethantechnologie bekannten Verbindungen. Bevorzugt sind hier Verbindungen der Gruppe bestehend aus katalytisch aktiven Metallsalzen, Aminen, Amidinen und Guanidinen. Beispielhaft zu nennen sind Zinndibutyldilaurat (DBTL), Zinnoctoat (SO), Zinnacetat, Zinkoctoat (ZO), 1,8-Diazabi-cyclo[5.4.0]undecen-7 (DBU), 1,5-Diazabicyclo[4.3.0]nonen-5 (DBN), 1,4-Diazabicy-clo[3.3.0]octen-4 (DBO), N-Ethylmorpholin (NEM), Triethylendiamin (DABCO), Pentamethylguanidin (PMG), Tetrametylguanidin (TMG), Cyclotetramethylguanidin (TMGC), n-Decyl-tetramethylguanidin (TMGD), n-Dodecyltetramethylguanidin (TMGDO), Dimethylaminoethyltetramethylguanidin (TMGN), 1,1,4,4,5,5-Hexamethylisobiguanidin (HMIB), Phenyltetramethylguanidin (TMGP) und Hexamethylenoctamethylbiguanidin (HOBG).

Besonders bevorzugt ist allerdings, wenn keine Katalysatoren E) eingesetzt werden.

.Zur Verbesserung der Schaumbildung, Schaumstabilität oder der Eigenschaften des resultierenden Polyurethan-Schaums können Tenside F) zugesetzt werden, wobei solche Additive grundsätzlich alle an sich bekannten anionischen, kationischen, amphoteren und nichtionischen Tenside sowie Mischungen hieraus sein können. Bevorzugt werden Alkylpolyglycoside, EO/PO-Blockcopolymere, Alkyl- oder Arylalkoxylate, Siloxanalkoxylate, Ester der Sulfobernsteinsäure und/oder Alkali- oder Erdalkalimetallalkanoate verwendet. Besonders bevorzugt werden EO/PO-Blockcopolymere und ganz besonders bevorzugt ausschließlich EO/PO-Blockcopolymere als Tenside F) eingesetzt.

Zudem können zur Verbesserung der Eigenschaften des resultierenden Polyurethan-Schaums ein- und mehrwertigen Alkohole G) sowie Mischungen hieraus eingesetzt werden. Beispiele sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole.

Ebenfalls optional können hydrophile Polyisocyanate H) mit umgesetzt werden. Bei der Herstellung der hydrophilen Polyisocyanate H) wird das Verhältnis der monofunktionellen Polyalkylenoxide H2) zu den niedermolekularen, aliphatischen Diisocyanaten H1) typischerweise so eingestellt, dass auf 1 Mol OH-Gruppen der monofunktionellen Polyalkylenoxide 1,25 bis 15 Mol, bevorzugt 2 bis 10 Mol und besonders bevorzugt 2 bis 6 Mol NCO-Gruppen des niedermolekularen, aliphatischen Diisocyanats H1) kommen. Anschließend erfolgt die Allophanatisierung bzw. Biurethisierung und/oder Isocyanuratbildung bzw. Uretdionbildung. Werden die Polyalkylenoxide H2) über Urethangruppen an die aliphatischen Diisocyanate H1) gebunden, findet bevorzugt im Anschluss eine Allophanatisierung statt. Weiterhin ist bevorzugt, dass Isocyanurat-Struktureinheiten gebildet werden.

Eine alternative Herstellung der hydrophilen Polyisocyanate H) erfolgt typischerweise durch Umsetzung von 1 Mol OH-Gruppen der monofunktionellen Polyalkylenoxidkomponente H2) mit 1,25 bis 15 Mol, bevorzugt 2 bis 10 Mol und besonders bevorzugt 2 bis 6 Mol NCO-Gruppen eines Polyisocyanates H1) mit einer Isocyanatfunktionalität von 2 bis 6, dass auf aliphatischen Disocyanaten basiert. Beispielhaft für derartige Polyisocyanate H1) sind Biuret-Strukturen, Isocyanurate bzw. Uretdione basierend auf aliphatischen Diisocyanaten. Dabei werden die Polyisocyanate H1) und die Polyalkylenoxide H2) bevorzugt über eine Urethangruppe bzw. eine Harnstoffgruppe miteinander verknüpft, wobei besonders die Verknüpfung über Urethangruppen bevorzugt ist.

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung erfolgt typischerweise bei 25 bis 140 °C, bevorzugt 60 bis 100 °C.

Wurde mit überschüssigem niedermolekularen Diisocyanat gearbeitet, erfolgt anschließend die Entfernung des Überschusses an niedermolekularem, aliphatischen Diisocyanat bevorzugt durch Dünnschichtdestillation.

Vor, während und nach der Reaktion oder der destillativen Abtrennung des Diisocyanatüberschusses können saure oder alkylierende Stabilisatoren, wie Benzoylchlorid, Isophthaloylchlorid, Methyltosylat, Chlorpropionsäure, HCl oder Antioxidantien, wie Di-*tert*-butylkresol oder Tocopherol zugesetzt werden.

Der NCO-Gehalt (bestimmt nach DIN-EN ISO 11909) der hydrophilen Polyisocyanate H) beträgt bevorzugt 0,3 bis 20 Gew.-%, besonders bevorzugt 2 bis 10 Gew.-% und ganz besonders bevorzugt 3 bis 6 Gew.%.

Beispiele für niedermolekulare, aliphatische Diisocyanate der Komponente H1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat, Isophorondiisocyanat, Butylendiisocyanat und Bis(isocyanatocyclohexyl)methan bevorzugt sind. Besonders bevorzugt sind Hexamethylendiisocyanat, Isophorondiisocyanat, Butylendiisocyanat und ganz besonders bevorzugt Hexamethylendiisocyanat und Isophorondiisocyanat.

Beispiele für höhermolekulare Polyisocyanate H1) sind Polyisocyanate mit einer Isocyanatfunktionalität von 2 bis 6 mit Isocyanurat-, Urethan-, Allophanat-, Biuret-, Iminooxadiazintrion-, Oxadiazintrion- und/oder Uretdiongruppen auf Basis der im vorstehenden Abschnitt genannten aliphatischen und/oder cycloaliphatischen Diisocyanate.

Bevorzugt werden als Komponente H1) höhermolekulare Verbindungen mit Biuret-, Iminooxadiazindion,- Isocyanurat- und/oder Uretdiongruppen auf Basis von Hexamethylendiisocyanat, Isophorondiisocyanat und/oder 4,4'-Diisocyanatodicyclohexylmethan eingesetzt. Weiter bevorzugt sind Isocyanurate. Ganz besonders bevorzugt sind Strukturen auf Basis von Hexamethylendiisocyanat.

Die Herstellung von Polyalkylenoxiden H2) durch Alkoxylierung geeigneter Startermoleküle ist literaturbekannt (z.B. Ullmanns Encyclopädie der technischen Chemie, 4. Auflage, Band 19, Verlag Chemie, Weinheim S. 31-38). Geeignete Startermoleküle sind insbesondere gesättigte Monoalkohole wie Methanol, Ethanol, n-Propanol, Isopropanol, n-Butanol, Isobutanol, sec-Butanol, Diethylenglykolmonobutylether sowie aromatische Alkohole wie Phenol oder Monoamine wie Diethylamin. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Unter monofunktionellen Polyalkylenoxiden im Sinne der Erfindung sind Verbindungen zu verstehen, die nur eine isocyanatreaktive Gruppe, d.h. eine Gruppe, die mit einer NCO-Gruppe reagieren kann, aufweisen.

Die monofunktionellen Polyalkylenoxide H2) besitzen bevorzugt eine OH-Gruppe als isocyanatreaktive Gruppe.

Die monofunktionellen Polyalkylenoxiden H2) weisen eine OH-Zahl von 15 bis 250, bevorzugt von 28 bis 112, und einem Ethylenoxidanteil von 50 bis 100 mol%, bevorzugt von 60 bis 100 mol%, bezogen auf die Gesamtmenge der enthaltenen Oxyalkylengruppen auf.

Die monofunktionellen Polyalkylenoxide H2) besitzen typischerweise zahlenmittlere Molekulargewichte von 220 bis 3700 g/mol, bevorzugt von 500 bis 2800 g/mol.

Gemäß einer weiteren bevorzugten Ausführungsform der Erfindung ist vorgesehen, dass die Komponenten A) bis H) in folgenden Mengen eingesetzt werden:
A) 100 Gewichtsteile isocyanatfunktioneller Präpolymere,
B) 0,01 bis 5 Gewichtteile Alkalimetallsalze schwacher anorganischer Säuren,
C) 0,1 bis 200 Gewichtsteile Wasser,
D) 0 bis 100 Gewichtsteile heterocyclischer Oligomere,
E) 0 bis 1 Gewichtteile Katalysatoren,
F) 0 bis 10 Gewichtsteile Tenside,
G) 0 bis 20 Gewichtsteile ein- oder mehrwertige Alkohole und
H) 0 bis 60 Gewichtsteile hydrophile Polyisocyanate.

Vorteilhaft ist auch, wenn 10 bis 100, bevorzugt 20 bis 90 und weiter bevorzugt 20 bis 80 Gewichtsteile heterocyclische Oligomere D) eingesetzt werden.

Die Herstellung der erfindungsgemäßen hydrophilen, aliphatischen Polyurethan-Schäume kann durch Mischen der Komponenten A), B), C), und gegebenenfalls D), E), F), G) und H)in beliebiger Reihenfolge, Aufschäumen der Mischung und Aushärtung, bevorzugt durch chemische Vernetzung, erfolgen. Bevorzugt werden die Komponenten A), D) und ggf. H) miteinander vorvermischt. Die Komponenten B) wird bevorzugt in Form ihrer wässrigen Lösungen dem Reaktionsgemisch zugesetzt.

Das Aufschäumen kann dabei grundsätzlich durch das bei der Reaktion der Isocyanatgruppen mit Wasser gebildete Kohlendioxid erfolgen, die Verwendung von weiteren Treibmitteln ist jedoch ebenfalls möglich. So können prinzipiell auch Treibmittel aus der Klasse der Kohlenwasserstoffe wie C₃C₆-Alkane, z.B. Butane, *n*-Pentan, *iso*-Pentan, *cyclo*-Pentan, Hexane o.ä. oder halogenierte Kohlenwasserstoffe wie Dichlormethan, Dichlormonofluormethan, Chlordifluorethane, 1,1-Dichlor-2,2,2Trifluorethan, 2,2-Dichlor-2-fluorethan, insbesondere chlorfreie Fluorkohlenwasserstoffe wie Difluormethan, Trifluormethan, Difluorethan, 1,1,1,2-Tetrafluorethan, Tetrafluorethan (R 134 oder R 134a), 1,1,1,3,3-Pentafluorpropan (R 245 fa), 1,1,1,3,3,3-Hexafluorpropan (R 256), 1,1,1,3,3-Pentafluorbutan (R 365 mfc), Heptafluorpropan oder auch Schwefelhexafluorid verwendet werden. Auch Gemische dieser Treibmittel sind verwendbar.

Die anschließende Aushärtung erfolgt typischerweise bei Raumtemperatur.

Ein weiterer Gegenstand der vorliegenden Erfindung sind Zusammensetzungen umfassend
A) isocyanatfunktionelle Präpolymere, bevorzugt mit einem Gewichtsanteil an niedermolekularen, aliphatischen Diisocyanaten mit einer Molmasse von 140 bis 278 g/mol von unter 1,0 Gew.-%, bezogen auf das Präpolymer, erhältlich durch Umsetzung von
   A1) niedermolekularen, aliphatischen Diisocyanaten einer Molmasse von 140 bis 278 g/mol mit
   A2) di- bis hexafunktionellen, bevorzugt tri- bis hexafunktionellen Polyalkylenoxiden einer OH-Zahl von 22,5 bis 112, bevorzugt von 31,5 bis 56, und einem Ethylenoxidanteil von 50 bis 100 mol%, bevorzugt von 60 bis 85 mol%, bezogen auf die Gesamtmenge der enthaltenen Oxyalkylengruppen,
B) Alkalimetallsalze schwacher anorganischer Säuren,
C) Wasser
D) gegebenenfalls heterocyclische, 4-Ring- oder 6-Ring-Oligomere von niedermolekularen, aliphatischen Diisocyanaten mit einer Molmasse von 140 bis 278 g/mol,
E) gegebenenfalls Katalysatoren,
F) gegebenenfalls Tenside,
G) gegebenenfalls ein- oder mehrwertige Alkohole und
H) gegebenenfalls hydrophile Polyisocyanate erhältlich durch Umsetzung von
   H1) niedermolekularen, aliphatischen Diisocyanaten einer Molmasse von 140 bis 278 g/mol und / oder daraus herstellbaren Polyisocyanaten mit einer Isocyanatfunktionalität von 2 bis 6 mit
   H2) monofunktionellen Polyalkylenoxiden einer OH-Zahl von 10 bis 250 und einem Ethylenoxidanteil von 50 bis 100 mol%, bezogen auf die Gesamtmenge der enthaltenen Oxyalkylengruppen,
wobei die Alkalimetallsalze schwacher anorganischer Säuren B) Verbindungen sind, deren korrespondierende freie Säuren in Wasser bei 25°C einen pKS-Wert von ≥ 4,0 aufweisen.

Die erfindungsgemäßen Zusammensetzungen eignen sich insbesondere zur Verwendung in einem Verfahren zur Herstellung von Polyurethan-Schäumen.

Gemäß einer bevorzugten Ausführungsform der erfindungsgemäßen Zusammensetzungen sind die Alkalimetallsalze schwacher anorganischer Säuren B) Verbindungen, deren korrespondierende freie Säuren in Wasser bei 25°C einen pKS-Wert von ≥ 4,0 und ≤ 14 aufweisen.

Die Alkalimetallsalze schwacher anorganischer Säuren B) können besonders bevorzugt aus der Gruppe Natriumhydroxid, Natriumhydrogencarbonat und Natriumcarbonat ausgewählt sein.

Weiter bevorzugt sind auch Zusammensetzungen, bei denen die Komponenten A) bis H) in folgenden Mengen enthalten sind:
A) 100 Gewichtsteile isocyanatfunktioneller Präpolymere,
B) 0,01 bis 5 Gewichtsteile Alkalimetallsalze schwacher anorganischer Säuren,
C) 0,1 bis 200 Gewichtsteile Wasser,
D) 0 bis 100 Gewichtsteile heterocyclischer Oligomere,
E) 0 bis 1 Gewichtteile Katalysatoren,
F) 0 bis 10 Gewichtsteile Tenside,
G) 0 bis 20 Gewichtsteile ein- oder mehrwertige Alkohole und
H) 0 bis 60 Gewichtsteile hydrophile Polyisocyanate.

Vorteilhafterweise enthalten die Zusammensetzungen 1 bis 100, bevorzugt 5 bis 90, weiter bevorzugt 10 bis 80 Gewichtsteile der heterocyclischen Oligomere D).

Nach der Herstellung können die Polyurethan-Schäume nach an sich bekannten Verfahren zu flächigen Materialien verarbeitet werden, welche dann beispielsweise als Bestandteil einer Wundauflage, eines kosmetischen Artikels oder eines Inkontinenzprodukts eingesetzt werden. In der Regel werden dazu Blockschäume auf die gewünschte Dicke nach gängigen Methoden geschnitten, um so flächige Materialien mit einer Dicke von typischerweise 10 µ bis 5 cm, bevorzugt 0,1 mm bis 1 cm, besonders bevorzugt 0,1 mm bis 6 mm, ganz besonders bevorzugt 0,2 mm bis 6 mm zu erhalten.

Durch geeignete Gießtechniken können die beschriebenen flächigen Materialien aber auch direkt durch Auftrag und Aufschäumen der erfindungsgemäßen Zusammensetzung auf ein Substrat, z.B. ein gegebenenfalls vorbehandeltes Papier oder Textil, erhalten werden.

Die Polyurethan-Schäume können überdies mit weiteren Materialien, beispielsweise auf Basis von Hydrogelen, (semi-) permeablen Folien, Schaumfolien, Beschichtungen, Hydrokolloiden oder anderen Schäumen verklebt, laminiert oder beschichtet werden.

Die Polyurethan-Schäume sind besonders zur Herstellung von Wundauflagen geeignet. Dabei können die Polyurethan-Schäume in direktem oder indirektem Kontakt mit der Wunde sein. Bevorzugt werden die Polyurethan-Schäume jedoch in direktem Kontakt mit der Wunde eingesetzt, um beispielsweise eine optimale Absorption von Wundflüssigkeit zu gewährleisten. Die Polyurethan-Schäume zeigen keine Zelltoxizität (Bestimmung nach ISO 10993-5 und ISO 1099312).

Die Polyurethan-Schäume, die als Wundauflage eingesetzt werden, können zusätzlich noch in einem weiteren Verfahrensschritt sterilisiert werden. Zur Sterilisation können die dem Fachmann an sich bekannten Verfahren zum Einsatz kommen, bei denen eine Sterilisation durch thermische Behandlung, chemische Stoffe wie Ethylenoxid oder Bestrahlung beispielsweise durch Gammabestrahlung erfolgt. Die Bestrahlung kann dabei gegebenenfalls in einer Schutzgasatmosphäre erfolgen. Die erfindungsgemäßen Polyurethan-Schäume haben dabei den großen Vorteil, dass sie sich bei Bestrahlung, insbesondere bei Bestrahlung mit Gammastrahlen, nicht verfärben.

Ebenfalls möglich ist die Zugabe, Einarbeitung oder Beschichtung von bzw. mit antimikrobiellen oder biologischen Wirkstoffen, welche sich beispielsweise in Bezug auf die Wundheilung und die Vermeidung von Keimbelastungen positiv auswirken.

### Beispiele

Die Erfindung wir im Folgenden anhand von Beispielen näher erläutert.

### Verwendete Substanzen und Abkürzungen:

| | |
|---|---|
| Desmodur^{®} N 3400: | Aliphatisches Polyisocyanat (HDI-Uretdion), NCO-Gehalt 21,8 %, Bayer MaterialScience AG, Leverkusen, Deutschland |
| Desmodur^{®} N 3300: | Aliphatisches Polyisocyanat (HDI-Isocyanurat), NCO-Gehalt 21,8 %, Bayer MaterialScience AG, Leverkusen, Deutschland |
| Polyether LB 25: | monofunktioneller Polyether auf Ethylenoxid-/Propylenoxidbasis, zahlenmittleres Molekulargewicht 2250 g/mol, OH-Zahl 25 mg KOH/g , Bayer MaterialScience AG, Leverkusen, Deutschland |

### Methoden:

Sofern nicht abweichend gekennzeichnet, beziehen sich alle Prozentangaben auf das Gewicht.

Die Bestimmung der Festkörpergehalte erfolgte nach DIN-EN ISO 3251.

Die Bestimmung der Viskositäten erfolgte bei 23 °C und wurde nach DIN 53019 durchgeführt.

Die NCO-Gehalte wurden volumetrisch gemäß DIN-EN ISO 11909 bestimmt.

### Beispiel 1: Herstellung des Polyurethan-Präpolymers 1 (Komponente A)

Zu einem Gemisch aus 1000 g HDI und 1 g Benzoylchlorid wurde bei 80 °C innerhalb von 3 h 1000 g eines Polyalkylenoxids mit einer Molmasse von 4680 g/mol, gestartet auf Glycerin, einem Ethylenoxidgewichtsanteil von 72 % und einen Propylenoxidgewichtsanteil von 28 %, das zuvor bei 100 °C während 6 h bei einem Druck von 0,1 mbar getrocknet wurde, zugetropft und für 12 h nachgeführt. Das überschüssige HDI wurde durch Dünnschichtdestillation bei 130 °C und 0,1 mbar entfernt, wobei die nicht flüchtigen Bestandteile mit 1 g Chlorpropionsäure stabilisiert wurden. Man erhielt ein Präpolymer A) mit einem NCO-Gehalt von 2,77 % und einer Viskosität von 3500 mPas.

### Beispiel 2: Herstellung eines hydrophilen Polyisocyanates (Komponente H)

Ein Gemisch aus 282,5 g Desmodur N 3300 und 843,8 g eines Mono-Hydroxy-funktionellen Polyethers auf Ethylenoxid-/Propylenoxidbasis (mit einem Ethylenoxid-Anteil von 80 mol, bezogen auf die Gesamtmenge der enthaltenen Oxyalkylengruppen, mit einem zahlenmittleren Molekulargewicht von 2250 g/mol und einer OH-Zahl von 25 mg KOH/g wurde in einer Glasapparatur so lang bei 80 °C gerührt, bis der tritrimetrisch ermittelte Gehalt an NCO-Gruppen konstant war. Man erhielt eine Flüssigkeit mit einem NCO-Gehalt von 4,04 % und einer Viskosität von 3330 mPas.

### Herstellung von Schaumstoffen

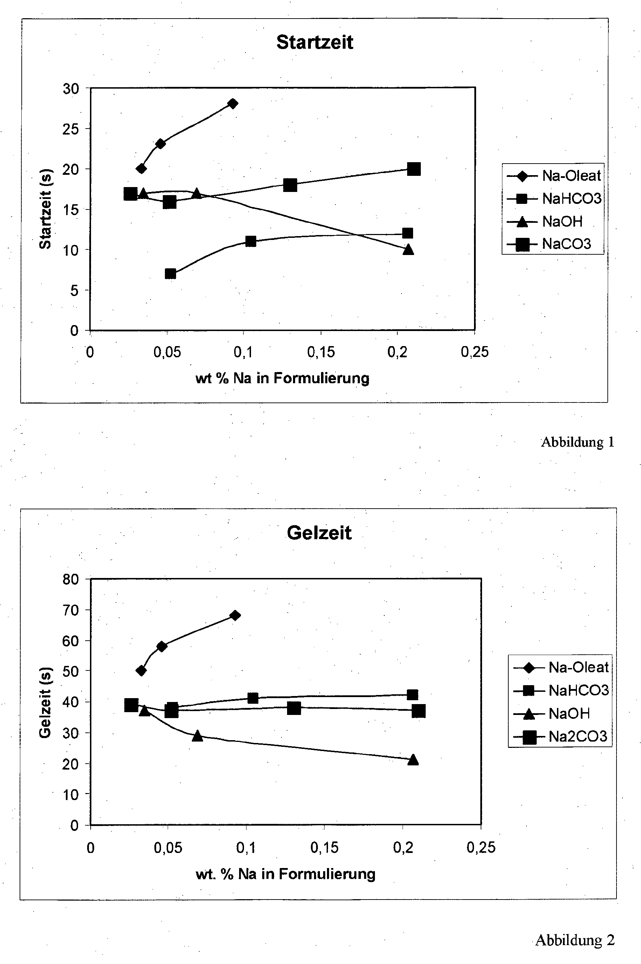

Die Art und Menge der hergestellten und aufgeschäumten Zusammensetzungen sind Tabelle 1 und den Abbildungen 1 und 2 zu entnehmen. Die Herstellung der Zusammensetzungen erfolgte wie folgt: Das Präpolymer aus Beispiel 1, das hydrophile Polyisocyanate aus Beispiel 2 und das Oligomer Desmodur N3400 wurden in ein Gefäß gefüllt und dort für 15 Sekunden mit einer Drehzahl von 1200 Upm durch Rühren homogenisiert. Anschließend wurde Wasser mit der jeweiligen, bereits darin gelösten Menge der Alkalisalze schwacher Säuren (Mengen gemäß Abbildungen 1 und 2) zugegeben und dann weitere 10 Sekunden gerührt. Das so erhaltene Zusammensetzungen wurden dann stehen gelassen, bis sich durch Reaktion der Komponenten ein stabiler Schaum gebildet hatte.

Während des Aufschäumens wurden die Startzeit und Gelzeit bestimmt. Die Startzeit war dabei der Zeitpunkt, zu dem der Beginn der Reaktion der Komponenten beobachtet werden konnte, was an der Entstehung erster Gasbläschen festgemacht wurde. Als Gelzeit wurde der Zeitpunkt bestimmt, zu dem die Zusammensetzung begann Fäden zu ziehen. Dies wurde geprüft, indem wiederholt eine Kunststoffpipette kurz mit der Zusammensetzung in Kontakt gebracht und anschließend weggezogen wurde. Sobald es hierbei nicht mehr zu einer Fadenbildung zwischen der Zusammensetzung und der Kunststoffpipette kam, war die Gelzeit erreicht..

**Tabelle 1**

| Komponente | Gewichtsteile |
|---|---|
| Präpolymer A) aus Beispiel 1 | 180 |
| Oligomer D) | 25 |
| Hydrophiles PIC H) aus Beispiel 2 | 45 |
| Wässrige Phase: | 34 |
| Komponente B) gemäß den in den Abbildungen 1 und 2 dargestellten Mengen jeweils mit der entsprechenden Wasser ergänzt | |

In den Abbildungen 1 und 2 sind jeweils für unterschiedliche Alkalisalze schwacher Säuren die Start- und die Gelzeit abhängig von der Natriumkonzentration in der Formulierung und von dem Typ des Natriumsalzes dargestellt.

Neben den erfindungsgemäßen Polyurethan-Schäumen, die jeweils ein Alkalisalz einer schwachen anorganischen Säure enthielten (NaHCO₃, NaOH und Na₂CO₃) wurde als Gegenbeispiel auch Schäume unter Verwendung eines Alkalisalzes einer schwachen organischen Säure (Natriumoleat) hergestellt.

Beim Aufschäumen hat sich gezeigt, dass die erfindungsgemäßen Zusammensetzungen enthaltend NaHCO₃, NaOH oder Na₂CO₃, einerseits deutlich kürzere Start- und Gelzeiten und damit kürzere Reaktionszeiten als die Vergleichszusammensetzungen enthaltend Natriumoleat hatten. Andererseits unterschieden sich die aus den erfindungsgemäßen Zusammensetzungen hergestellten Schäume in ihren chemischen wie physikalischen Eigenschaften nicht von den Schäumen aus den Vergleichszusammensetzungen. So weisen die erfindungsgemäßen Polyurethan-Schäume ein großes Absorptionsvermögen für physiologische Salzlösung bzw. für Wundflüssigkeit und nur einen kleinen Anteil an extrahierbaren Bestandteilen auf. Außerdem sind die Schäume sehr gut zellverträglich..

## Patentansprüche

1. Verfahren zur Herstellung hydrophiler, aliphatischer Polyurethan-Schäume, bei dem Zusammensetzungen umfassend
A) isocyanatfunktionelle Präpolymere erhältlich durch Umsetzung von
A1) niedermolekularen, aliphatischen Diisocyanaten einer Molmasse von 140 bis 278 g/mol mit
A2) di- bis hexafunktionellen Polyalkylenoxiden einer OH-Zahl von 22,5 bis 112 mg KOH/g und einem Ethylenoxidanteil von 50 bis 100 mol%, bezogen auf die Gesamtmenge der enthaltenen Oxyalkylengruppen,
B) Alkalimetallsalze schwacher anorganischer Säuren,
C) Wasser
D) gegebenenfalls heterocyclische 4-Ring- oder 6-Ring-Oligomere von niedermolekularen, aliphatischen Diisocyanaten mit einer Molmasse von 140 bis 278 g/mol,
E) gegebenenfalls Katalysatoren,
F) gegebenenfalls Tenside,
G) gegebenenfalls ein- oder mehrwertige Alkohole und
H) gegebenenfalls hydrophile Polyisocyanate erhältlich durch Umsetzung von
H1) niedermolekularen, aliphatischen Diisocyanaten einer Molmasse von 140 bis 278 g/mol und / oder daraus herstellbaren Polyisocyanaten mit einer Isocyanatfunktionalität von 2 bis 6 mit
H2) monofunktionellen Polyalkylenoxiden einer OH-Zahl von 10 bis 250 und einem Ethylenoxidanteil von 50 bis 100 mol%, bezogen auf die Gesamtmenge der enthaltenen Oxyalkylengruppen,
bereitgestellt, aufgeschäumt und ausgehärtet werden, wobei Alkalimetallsalze schwacher anorganischer Säuren B) verwendet werden, deren korrespondierende freie Säuren in Wasser bei 25 °C einen pKs-Wert von ≥ 4,0 aufweisen.

2. Verfahren nach Anspruch 1, **dadurch gekennzeichnet, dass** die Alkalimetallsalze schwacher anorganischer Säuren B) aus der Gruppe Natriumhydroxid, Natriumhydrogencarbonat und Natriumcarbonat ausgewählt sind.

3. Verfahren nach einem der Ansprüche 1 oder 2, **dadurch gekennzeichnet, dass** isocyanatfunktionelle Präpolymere A) mit einem Gewichtsanteil an niedermolekularen, aliphatischen Diisocyanaten mit einer Molmasse von 140 bis 278 g/mol von unter 1,0 Gew.-%, bezogen auf das Präpolymer, verwendet werden.

4. Verfahren nach einem der Ansprüche 1 bis 3, **dadurch gekennzeichnet, dass** die Komponenten A) bis H) in folgenden Mengen eingesetzt werden:
A) 100 Gewichtsteile isocyanatfunktioneller Präpolymere,
B) 0,01 bis 5 Gewichtteile Alkalimetallsalze schwacher anorganischer Säuren,
C) 0,1 bis 200 Gewichtsteile Wasser,
D) 0 bis 100 Gewichtsteile heterocyclischer Oligomere,
E) 0 bis 1 Gewichtteile Katalysatoren,
F) 0 bis 10 Gewichtsteile Tenside,
G) 0 bis 20 Gewichtsteile ein- oder mehrwertige Alkohole und
H) 0 bis 60 Gewichtsteile hydrophile Polyisocyanate.

5. Verfahren nach einem der Ansprüche 1 bis 4, **dadurch gekennzeichnet, dass** 10 bis 100, bevorzugt 20 bis 90, weiter bevorzugt 20 bis 80, Gewichtsteile heterocyclische Oligomere D) eingesetzt werden.

6. Verfahren nach einem der Ansprüche 1 bis 5, **dadurch gekennzeichnet, dass** als niedermolekulare, aliphatische Diisocyanate A1) ausschließlich Hexamethylendiisocyanat (HDI), Isophorondiisocyanat (IPDI) oder deren Mischungen eingesetzt werden.

7. Verfahren nach einem der Ansprüche 1 bis 6 **dadurch gekennzeichnet, dass** als Polyalkylenoxide A2) Copolymere aus Ethylenoxid und Propylenoxid mit einem Ethylenoxidgehalt, bezogen auf die Gesamtmenge der enthaltenen Oxyalkylengruppen, von 60 bis 85 mol%, gestartet auf Polyolen oder Aminen eingesetzt werden.

8. Verfahren nach einem der Ansprüche 1 bis 7, **dadurch gekennzeichnet, dass** die Polyalkylenoxide A2) zahlenmittlere Molekulargewichte von 3000 bis 8500 g/mol aufweisen.

9. Verfahren nach einem der Ansprüche 1 bis 8, **dadurch gekennzeichnet, dass** die Polyalkylenoxide A2) OH-Funktionalitäten von 3 bis 4 aufweisen.

10. Verfahren nach einem der Ansprüche 1 bis 9, **dadurch gekennzeichnet, dass** keine Katalysatoren E) eingesetzt werden.

11. Zusammensetzungen umfassend
A) isocyanatfunktionelle Präpolymere, bevorzugt mit einem Gewichtsanteil an niedermolekularen, aliphatischen Diisocyanaten mit einer Molmasse von 140 bis 278 g/mol von unter 1,0 Gew.-%, bezogen auf das Präpolymer, erhältlich durch Umsetzung von
A1) niedermolekularen, aliphatischen Diisocyanaten einer Molmasse von 140 bis 278 g/mol mit
A2) di- bis hexafunktionellen, bevorzugt tri- bis hexafunktionellen Polyalkylenoxiden einer OH-Zahl von 22,5 bis 112, bevorzugt von 31,5 bis 56, und einem Ethylenoxidanteil von 50 bis 100 mol%, bevorzugt von 60 bis 85 mol%, bezogen auf die Gesamtmenge der enthaltenen Oxyalkylengruppen,
B) Alkalimetallsalze schwacher anorganischer Säuren,
C) Wasser
D) gegebenenfalls heterocyclische, 4-Ring- oder 6-Ring-Oligomere von niedermolekularen, aliphatischen Diisocyanaten mit einer Molmasse von 140 bis 278 g/mol,
E) gegebenenfalls Katalysatoren,
F) gegebenenfalls Tenside,
G) gegebenenfalls ein- oder mehrwertige Alkohole und
H) gegebenenfalls hydrophile Polyisocyanate erhältlich durch Umsetzung von
H1) niedermolekularen, aliphatischen Diisocyanaten einer Molmasse von 140 bis 278 g/mol und / oder daraus herstellbaren Polyisocyanaten mit einer Isocyanatfunktionalität von 2 bis 6 mit
H2) monofunktionellen Polyalkylenoxiden einer OH-Zahl von 10 bis 250 und einem Ethylenoxidanteil von 50 bis 100 mol%, bezogen auf die Gesamtmenge der enthaltenen Oxyalkylengruppen,
wobei die Alkalimetallsalze schwacher anorganischer Säuren B) Verbindungen sind, deren korrespondierende freie Säuren in Wasser bei 25°C einen pKS-Wert von ≥ 4,0 aufweisen.

12. Zusammensetzungen nach Anspruch 11, **dadurch gekennzeichnet, dass** die Alkalimetallsalze schwacher anorganischer Säuren B) aus der Gruppe Natriumhydroxid, Natriumhydrogencarbonat und Natriumcarbonat ausgewählt sind.

13. Zusammensetzungen nach Anspruch 11 oder 12, **dadurch gekennzeichnet, dass** die Komponenten A) bis H) in folgenden Mengen in den Zusammensetzungen enthalten sind:
A) 100 Gewichtsteile isocyanatfunktioneller Präpolymere,
B) 0,01 bis 5 Gewichtsteile Alkalimetallsalze schwacher anorganischer Säuren,
C) 0,1 bis 200 Gewichtsteile Wasser,
D) 0 bis 100 Gewichtsteile heterocyclischer Oligomere,
E) 0 bis 1 Gewichtteile Katalysatoren,
F) 0 bis 10 Gewichtsteile Tenside,
G) 0 bis 20 Gewichtsteile ein- oder mehrwertige Alkohole und
H) 0 bis 60 Gewichtsteile hydrophile Polyisocyanate.

## Claims

1. Process for producing hydrophilic aliphatic polyurethane foams, which comprises compositions comprising
A) isocyanate-functional prepolymers obtainable by reaction of
A1) low molecular weight aliphatic diisocyanates having a molar mass of 140 to 278 g/mol with
A2) di- to hexafunctional polyalkylene oxides having an OH number of 22.5 to 112 mg KOH/g and an ethylene oxide content of 50 to 100 mol%, based on the total amount of oxyalkylene groups present,
B) alkali metal salts of weak inorganic acids,
C) water,
D) optionally heterocyclic 4-ring or 6-ring oligomers of low molecular weight aliphatic diisocyanates having a molar mass of 140 to 278 g/mol,
E) optionally catalysts,
F) optionally surfactants,
G) optionally mono- or polyhydric alcohols, and
H) optionally hydrophilic polyisocyanates obtainable by reaction of
H1) low molecular weight aliphatic diisocyanates having a molar mass of 140 to 278 g/ml and/or polyisocyanates obtainable therefrom with an isocyanate functionality of 2 to 6, with
H2) monofunctional polyalkylene oxides having an OH number of 10 to 250 and an ethylene oxide content of 50 to 100 mol%, based on the total amount of oxyalkylene groups present,
being provided, foamed and cured, wherein alkali metal salts of weak inorganic acids B) are used whose corresponding free acids have a pKₐ value of ≥ 4.0 in water at 25°C.

2. Process according to Claim 1, **characterized in that** the alkali metal salts of weak inorganic acids B) are selected from the group sodium hydroxide, sodium bicarbonate and sodium carbonate.

3. Process according to either Claim 1 or 2, **characterized in that** isocyanate-functional prepolymers A) having a weight fraction of below 1.0 wt% for low molecular weight aliphatic diisocyanates having a molar mass of 140 to 278 g/mol, based on the prepolymer, are used.

4. Process according to any one of Claims 1 to 3, **characterized in that** the components A) to H) are used in the following amounts:
A) 100 parts by weight of isocyanate-functional prepolymers,
B) 0.01 to 5 parts by weight of alkali metal salts of weak inorganic acids,
C) 0.1 to 200 parts by weight of water,
D) 0 to 100 parts by weight of heterocyclic oligomers,
E) 0 to 1 part by weight of catalysts,
F) 0 to 10 parts by weight of surfactants,
G) 0 to 20 parts by weight of mono- or polyhydric alcohols, and
H) 0 to 60 parts by weight of hydrophilic polyisocyanates.

5. Process according to any one of Claims 1 to 4, **characterized in that** 10 to 100, preferably 20 to 90 and more preferably 20 to 80 parts by weight of heterocyclic oligomers D) are used.

6. Process according to any one of Claims 1 to 5, **characterized in that** exclusively hexamethylene diisocyanate (HDI), isophorone diisocyanate (IPDI) or mixtures thereof are used as low molecular weight aliphatic diisocyanates A1).

7. Process according to any one of Claims 1 to 6, **characterized in that** copolymers of ethylene oxide and propylene oxide having an ethylene oxide content, based on the total amount of oxyalkylene groups present, of 60 to 85 mol%, and started on polyols or amines, are used as polyalkylene oxides A2).

8. Process according to any one of Claims 1 to 7, **characterized in that** the polyalkylene oxides A2) have number-average molecular weights of 3000 to 8500 g/mol.

9. Process according to any one of Claims 1 to 8, **characterized in that** the polyalkylene oxides A2) have OH functionalities of 3 to 4.

10. Process according to any one of Claims 1 to 9, **characterized in that** no catalysts E) are used.

11. Compositions comprising
A) isocyanate-functional prepolymers, preferably having a weight fraction of below 1.0 wt% for low molecular weight aliphatic diisocyanates having a molar mass of 140 to 278 g/mol, based on the prepolymer, obtainable by reaction of
A1) low molecular weight aliphatic diisocyanates having a molar mass of 140 to 278 g/mol with
A2) di- to hexafunctional, preferably tri-to hexafunctional, polyalkylene oxides having an OH number of 22.5 to 112, preferably 31.5 to 56, and an ethylene oxide content of 50 to 100 mol%, preferably of 60 to 85 mol%, based on the total amount of oxyalkylene groups present,
B) alkali metal salts of weak inorganic acids,
C) water,
D) optionally heterocyclic 4-ring or 6-ring oligomers of low molecular weight aliphatic diisocyanates having a molar mass of 140 to 278 g/mol,
E) optionally catalysts,
F) optionally surfactants,
G) optionally mono- or polyhydric alcohols, and
H) optionally hydrophilic polyisocyanates obtainable by reaction of
H1) low molecular weight aliphatic diisocyanates having a molar mass of 140 to 278 g/ml and/or polyisocyanates obtainable therefrom with an isocyanate functionality of 2 to 6, with
H2) monofunctional polyalkylene oxides having an OH number of 10 to 250 and an ethylene oxide content of 50 to 100 mol%, based on the total amount of oxyalkylene groups present,
wherein the alkali metal salts of weak inorganic acids B) are compounds whose corresponding free acids have a pKA value of ≥ 4.0 in water at 25°C.

12. Compositions according to Claim 11, **characterized in that** the alkali metal salts of weak inorganic acids B) are selected from the group sodium hydroxide, sodium bicarbonate and sodium carbonate.

13. Compositions according to Claim 11 or 12, **characterized in that** the components A) to H) are present in the composition in the following amounts:
A) 100 parts by weight of isocyanate-functional prepolymers,
B) 0.01 to 5 parts by weight of alkali metal salts of weak inorganic acids,
C) 0.1 to 200 parts by weight of water,
D) 0 to 100 parts by weight of heterocyclic oligomers,
E) 0 to 1 part by weight of catalysts,
F) 0 to 10 parts by weight of surfactants,
G) 0 to 20 parts by weight of mono- or polyhydric alcohols, and
H) 0 to 60 parts by weight of hydrophilic polyisocyanates.

## Revendications

1. Procédé pour la préparation de mousses de polyuréthane aliphatique, hydrophiles, dans lequel on prépare, fait mousser et durcit des compositions comprenant
A) des prépolymères à fonctionnalité isocyanate pouvant être obtenus par transformation
A1) de diisocyanates aliphatiques de bas poids moléculaire, présentant une masse molaire de 140 à 278 g/mole avec
A2) des poly(oxydes d'alkylène) difonctionnels à hexafonctionnels présentant un indice d'OH de 22,5 à 112 mg de KOH/g et une proportion d'oxyde d'éthylène de 50 à 100% en mole par rapport à la quantité totale des groupes d'oxyalkylène contenus,
B) des sels de métal alcalin d'acides inorganiques faibles,
C) de l'eau
D) le cas échéant des oligomères de 4 ou 6 cycles, hétérocycliques, de diisocyanates aliphatiques de bas poids moléculaire présentant une masse molaire de 140 à 278 g/mole,
E) le cas échéant des catalyseurs,
F) le cas échéant des agents tensioactifs,
G) le cas échéant des alcools monovalents ou polyvalents
H) le cas échéant des polyisocyanates hydrophiles pouvant être obtenus par transformation
H1) de diisocyanates aliphatiques de bas poids moléculaire, présentant une masse molaire de 140 à 278 g/mole et/ou de polyisocyanates pouvant être préparés à partir de ceux-ci présentant une fonctionnalité isocyanate de 2 à 6 avec
H2) des poly(oxydes d'alkylène) monofonctionnels présentant un indice d'OH de 10 à 250 et une proportion d'oxyde d'éthylène de 50 à 100% en mole par rapport à la quantité totale des groupes d'oxyalkylène contenus,
où on utilise des sels de métal alcalin d'acides inorganiques faibles B) dont les acides libres correspondants présentent, dans l'eau à 25°C, une valeur pKₐ ≥ 4,0.

2. Procédé selon la revendication 1, **caractérisé en ce que** les sels de métal alcalin d'acides inorganiques faibles B) sont choisis dans le groupe formé par l'hydroxyde de sodium, l'hydrogénocarbonate de sodium et le carbonate de sodium.

3. Procédé selon l'une quelconque des revendications 1 ou 2, **caractérisé en ce qu'**on utilise des prépolymères à fonctionnalité isocyanate A) présentant une proportion pondérale de diisocyanates aliphatiques de bas poids moléculaire, présentant une masse molaire de 140 à 278 g/mole, inférieure à 1,0% en poids par rapport au prépolymère.

4. Procédé selon l'une quelconque des revendications 1 à 3, **caractérisé en ce que** les composants A) à H) sont utilisés dans les quantités suivantes :
A) 100 parties en poids de prépolymères à fonctionnalité isocyanate,
B) 0,01 à 5 parties en poids de sels de métal alcalin d'acides inorganiques faibles,
C) 0,1 à 200 parties en poids d'eau,
D) 0 à 100 parties en poids d'oligomères hétérocycliques,
E) 0 à 1 partie en poids de catalyseurs,
F) 0 à 10 parties en poids d'agents tensioactifs,
G) 0 à 20 parties en poids d'alcools monovalents ou polyvalents et
H) 0 à 60 parties en poids de polyisocyanates hydrophiles.

5. Procédé selon l'une quelconque des revendications 1 à 4, **caractérisé en ce qu'**on utilise 10 à 100, de préférence 20 à 90, plus préférablement 20 à 80 parties en poids d'oligomères hétérocycliques D).

6. Procédé selon l'une quelconque des revendications 1 à 5, **caractérisé en ce qu'**on utilise, comme diisocyanates aliphatiques de bas poids moléculaire A1), exclusivement du diisocyanate d'hexaméthylène (HDI), du diisocyanate d'isophorone (IPDI) ou leurs mélanges.

7. Procédé selon l'une quelconque des revendications 1 à 6, **caractérisé en ce qu'**on utilise comme poly(oxydes d'alkylène) A2) des copolymères d'oxyde d'éthylène et d'oxyde de propylène présentant une teneur en oxyde d'éthylène, par rapport à la quantité totale des groupes d'oxyalkylène contenus, de 60 à 85% en mole, initiés sur des polyols ou des amines.

8. Procédé selon l'une quelconque des revendications 1 à 7, **caractérisé en ce que** les poly(oxydes d'alkylène) A2) présentent des poids moléculaires numériques moyens de 3000 à 8500 g/mole.

9. Procédé selon l'une quelconque des revendications 1 à 8, **caractérisé en ce que** les poly(oxydes d'alkylène) A2) présentent des fonctionnalités OH de 3 à 4.

10. Procédé selon l'une quelconque des revendications 1 à 9, **caractérisé en ce qu'**on n'utilise pas de catalyseurs E).

11. Compositions comprenant
A) des prépolymères à fonctionnalité isocyanate, présentant de préférence une proportion pondérale de diisocyanates aliphatiques de bas poids moléculaire, présentant une masse molaire de 140 à 278 g/mole, inférieure à 1,0% en poids par rapport au prépolymère, pouvant être obtenus par transformation
A1) de diisocyanates aliphatiques de bas poids moléculaire, présentant une masse molaire de 140 à 278 g/mole avec
A2) des poly(oxydes d'alkylène) difonctionnels à hexafonctionnels, de préférence trifonctionnels à hexafonctionnels, présentant un indice d'OH de 22,5 à 112, de préférence de 31,5 à 56, et une proportion d'oxyde d'éthylène de 50 à 100% en mole, de préférence de 60 à 85% en mole, par rapport à la quantité totale des groupes d'oxyalkylène contenus,
B) des sels de métal alcalin d'acides inorganiques faibles,
C) de l'eau
D) le cas échéant des oligomères de 4 ou 6 cycles, hétérocycliques, de diisocyanates aliphatiques de bas poids moléculaire présentant une masse molaire de 140 à 278 g/mole,
E) le cas échéant des catalyseurs,
F) le cas échéant des agents tensioactifs,
G) le cas échéant des alcools monovalents ou polyvalents
H) le cas échéant des polyisocyanates hydrophiles pouvant être obtenus par transformation
H1) de diisocyanates aliphatiques de bas poids moléculaire, présentant une masse molaire de 140 à 278 g/mole et/ou de polyisocyanates pouvant être préparés à partir de ceux-ci présentant une fonctionnalité isocyanate de 2 à 6 avec
H2) des poly(oxydes d'alkylène) monofonctionnels présentant un indice d'OH de 10 à 250 et une proportion d'oxyde d'éthylène de 50 à 100% en mole par rapport à la quantité totale des groupes d'oxyalkylène contenus,
les sels de métal alcalin d'acides inorganiques faibles B) étant des composés dont les acides libres correspondants présentent, dans l'eau à 25°C, une valeur pKₐ ≥ 4,0.

12. Compositions selon la revendication 11, **caractérisées en ce que** les sels de métal alcalin d'acides inorganiques faibles B) sont choisis dans le groupe formé par l'hydroxyde de sodium, l'hydrogénocarbonate de sodium et le carbonate de sodium.

13. Compositions selon la revendication 11 ou 12, **caractérisées en ce que** les composants A) à H) sont contenus dans les quantités suivantes dans les compositions :
A) 100 parties en poids de prépolymères à fonctionnalité isocyanate,
B) 0,01 à 5 parties en poids de sels de métal alcalin d'acides inorganiques faibles,
C) 0,1 à 200 parties en poids d'eau,
D) 0 à 100 parties en poids d'oligomères hétérocycliques,
E) 0 à 1 partie en poids de catalyseurs,
F) 0 à 10 parties en poids d'agents tensioactifs,
G) 0 à 20 parties en poids d'alcools monovalents ou polyvalents et
H) 0 à 60 parties en poids de polyisocyanates hydrophiles.
